# EUROPÄISCHE PATENTSCHRIFT

(11) **EP 0 605 527 B1**
(45) Veröffentlichungstag und Bekanntmachung des Hinweises auf die Patenterteilung: **22.01.1997**
(21) Anmeldenummer: 92919901.6
(22) Anmeldetag: 23.09.1992
(51) Int. Cl.: C12N 5/00

(54) **SUBSTRAT FÜR ZELLKULTUREN**
CELL CULTURE SUBSTRATE
SUBSTRAT POUR CULTURES DE CELLULES

(30) Priorität: 28.09.1991 DE 4132379
(43) Veröffentlichungstag der Anmeldung: 13.07.1994
(73) Patentinhaber: Forschungszentrum Karlsruhe GmbH, 76133 Karlsruhe (DE)
(72) Erfinder: WEIBEZAHN, Karl, Friedrich, D-7513 Stutensee 4 (DE); KNEDLITSCHEK, Gudrun, D-7500 Karlsruhe 41 (DE); DERTINGER, Hermann, D-6900 Heidelberg (DE); SCHUBERT, Klaus, D-7500 Karlsruhe 41 (DE); BIER, Wilhelm, D-7514 Eggenstein-Leopoldshafen (DE); SCHALLER, Thomas, D-7504 Weingarten (DE)
(74) Vertreter: Rückert, Friedrich, Dr.
(86) Internationale Anmeldenummer: DE9200815
(87) Internationale Veröffentlichungsnummer: WO9307258

(56) Entgegenhaltungen:
- EP-A- 0 014 007
- EP-A- 0 170 210
- EP-A- 0 205 790
- DE-C- 3 938 632
- FR-A- 2 522 014
- AMERICAN JOURNAL OF PHYSIOLOGY, Band 251, Nr. 1, 1986, US; R.E. STEELE et al., Seiten C136-C139

## Beschreibung

Die Erfindung betrifft ein Substrat für Zellkulturen bestehend aus
a) einem plattenförmigen Körper mit gitterartig angeordneten, gegeneinander durch Stege getrennten Durchbrüchen und
b) einem für Flüssigkeiten, nicht jedoch für Zellen durchlässigen Boden, der die Durchbrüche einseitig verschließt.

Ein solches Substrat ist aus der DE 29 02 026 Al bekannt. Dieses Substrat besteht aus einem Wandkörper und einem Boden, der mit dem Wandkörper lösbar verbunden ist. Der Wandkörper besteht aus einer Platte aus einem Kunststoff, die mehrere, durch Stege voneinander getrennte Durchbrüche (Kammern) aufweist. Aus den Figuren geht hervor, daß die lichte Weite der Durchbrüche etwa 10 mm (10000 µm) beträgt.
Als Boden werden Glas- oder vorzugsweise Kunststoffplatten bzw. Kunststoffolien eingesetzt. Bei diesem Substrat wachsen die Zellen auf dem Boden auf, weshalb sich mit diesem Substrat im wesentlichen zweidimensionale Zellkulturen ergeben. Es ist weiterhin angegeben, das der Boden mit der Zellkultur einen mikroskopischen Objektträger bilden oder zusätzlich auf einen Glasobjektträger aufgebracht werden kann. Auch hierbei wird davon ausgegangen, daß die Zellen praktisch nur am Boden anwachsen und hier eine im wesentlichen zweidimensionale Schicht ausbilden.

Aus der Veröffentlichung "Morphologische Untersuchungen an Eileiterepithelzellen des Kaninchens: Primärkulturen auf Polycarbonatmembranen" von I. G. Noske in TECNOMARA NEWS IV/90, Labortechnische Informationsschrift ist ein zweidimensionales Substrat für Zellkulturen und eine zweidimensionale Zellkultur bekannt. Das Substrat besteht aus einer Membran, die Poren unterschiedlicher Größe aufweist. Einige dieser Poren durchdringen die Membran. Die Zellen zeigen eine Tendenz, sich in diesen Poren zu verankern. Im wesentlichen bildet die Zellkultur eine Monoschicht, die die Oberseite und die Unterseite der Membran bedeckt.

Solche Substrate haben den Vorteil, daß Nährlösung den Zellen ungehindert zugeführt und Stoffwechselprodukte leicht abtransportiert werden können. Ferner besteht ebenfalls die Möglichkeit, auf solchen Substraten haftende Zellen optisch unter dem Mikroskop zu kontrollieren. Als Fazit der Veröffentlichung ist jedoch angeführt, daß die Zellen sich in Abhängigkeit vom angebotenen Substrat in ihrer Form verändern. So wachsen sie auf Glas- oder Plastikunterlage oft extrem flach, so daß die für ihre Funktion so wichtige Polarität nicht gewährleistet ist.

Aus den Veröffentlichungen "Solid tissue masses formed in vitro from cells cultured on artificial capillaries" von Richard A. Knazek, Federation Proceedings Vol. 33, No. 8 (August 1974) und einem Firmenprospekt der Firma «dunn Labortechnik GmbH» in Asbach, DE, mit dem Titel "Cellmax™ 100 - Hollow Fiber Bioreactor System for Mammalian and Insect Cell Culture", der auf die Veröffentlichung Knazek bezug nimmt, ist ein Zellsubstrat bekannt, das aus einer Vielzahl parallel verlaufender Kapillaren besteht, die im Abstand voneinander angeordnet sind. Die Kapillaren sind an ihren Enden durch jeweils ein Abschlußstück zusammengefasst, mit dem Nährlösung durch die Kapillaren geleitet werden kann. Die Zellen wachsen an der Außenseite der Kapillaren in dem Zwischenraum, den sie untereinander bilden, auf. Die Nährlösung diffundiert aus dem Innern der Kapillaren durch die Kapillarwand in den Zwischenraum und versorgt hier die Zellen. Auf diese Weise kann eine dreidimensionale Zellstruktur erzeugt werden.

Dieses Substrat erscheint aus verschiedenen Gründen zur Anlage und Untersuchung von Zellkulturen weniger geeignet. Der Stoffwechselgradient ist wegen der unregelmäßigen Geometrie nicht eindeutig definiert. Ferner wird die Diffusion von Produkten durch die Konstruktion der verschiedenen Kapillaren in Abhängigkeit vom verwendeten Material immer auf bestimmte Molekulargewichte eingeschränkt. Für manche Anwendungen ist eine Beschichtung des Substrats notwendig; eine solche Beschichtung kann bei diesem Zellsubstrat als Diffusionssperrschicht wirken und damit einen Gradienten zusammenbrechen lassen. Schließlich kann das bekannte Substrat nicht so ausgebildet werden, daß eine mikroskopische Kontrolle der Zellen während der Kultivierung möglich ist.

Ein weiteres Substrat für die Kultivierung von menschlichen und/oder tierischen Zellen ist aus der EP 0 205 790 B1 bekannt. Bei diesem Substrat handelt es sich um einen Verbund von perlenartigen Teilchen mit einem Durchmesser von etwa 50 bis 300 µm, in deren Zwischenräumen sich eine dreidimensionale Zellkultur ausbilden kann. Die perlenartigen Teilchen besitzen auf ihrer Oberfläche Makroporen, deren Durchmesser geringer als 10 µm ist und vorzugsweise im Bereich zwischen 0,1 und 3 µm liegt. Die Makroporen bilden ein Kapillarsystem, in welches die Zellen nicht hineinwachsen können, da der Zelldurchmesser der hierbei eingesetzten Zellen ca. 20 µm beträgt. Durch dieses Kapillarsystem erfolgt die Versorgung der Zellen mit Nährmedium und der Abtransport von Stoffwechselprodukten.

Dieses Substrat scheint mit ähnlichen Nachteilen behaftet zu sein wie das oben erwähnte Kapillarrohr-Substrat.

Ferner ist aus der FR-A 2 522 014 ein durch Abformung erhältliches, plattenförmiges Substrat bekannt, das eine Vielzahl von Vertiefungen mit einer Breite von 10 bis 1500 µm enthält. Dieses Substrat weist keinen für Flüssigkeiten durchlässigen Boden auf.

Der Erfindung liegt die Aufgabe zugrunde, den genannten Nachteilen der bekannten Zellsubstrate abzuhelfen. Insbesondere sollen die Vorteile von membranartigen Substraten mit der Möglichkeit, dreidimensionale Zellstrukturen ausbilden zu können, kombiniert werden.

Die Aufgabe wird bei dem eingangs genannten Substrat für Zellkulturen dadurch gelöst, daß die lichte Weite der Durchbrüche im Bereich zwischen 50 µm und 1000 µm liegt. Die abhängigen Ansprüche geben besonders vorteilhafte Ausführungsformen des Substrats an.

Die Durchbrüche können im Prinzip jede beliebige Form annehmen. Vorzugsweise besitzen sie eine Rechteck- oder Sechseckform, weil sich solche Formen mit Stegen von konstanter Breite realisieren lassen. Bei anderen, z. B. auch unregelmäßigen Formen soll die lichte Weite in jeder Richtung im angegebenen Bereich liegen. Die lichte Weite der Durchbrüche braucht nicht konstant zu sein. Die Durchbrüche können beispielsweise auch die Form von Rechteck- oder Sechseck-Pyramidenstümpfen, die sich in Richtung auf den Boden verjüngen, annehmen, wobei auch hier die angegebenen Werte für die lichte Weite in jeder Fläche parallel zum Boden eingehalten werden sollen.

Obwohl das erfindungsgemäße Substrat im wesentlichen die selbe äußere Form aufweist wie das aus der eingangs zitierten DE 29 02 026 Al, ergeben sich allein aus der Wahl anderer Abmessungen beträchtliche Unterschiede in der Wirkung des Substrats. Die Abmessungen des erfindungsgemäßen Substrats sind in der Weise gewählt, daß eingebrachte Zeilen nicht (wie in dem bekannten, eingangs beschriebenen Substrat) am Boden anwachsen, sondern sich an den seitlichen Wänden verankern und untereinander eine große Zahl von Verbindungen in horizontaler als auch besonders in vertikaler Richtung aufnehmen. Damit wird ein kompakter dreidimensionaler Zellverband aufgebaut, dessen Größe durch die Abmessung des einzelnen Durchbruchs vorgegeben ist. Aufgrund der erfindungsgemäß gewählten Größe wird zugleich die Versorgung des Zellverbands gewährleistet.

Der wesentliche Grundgedanke bei der Wahl der erfindungsgemäßen Abmessungen besteht darin, den einzelnen Zellen bereits bei der Anlage der Kultur möglichst wenig ebene Bodenfläche anzubieten. Es hat sich gezeigt, daß ein dreidimensionaler Aufbau einer Gewebestruktur nicht mehr erreicht werden kann, wenn sich wie beim eingangs beschriebenen Substrat mehrere Zellen unter Ausbildung einer zweidimensionalen Schicht am Boden anheften.

Besonders bevorzugt wird ein Substrat, bei dem die lichte Weite der Durchbrüche im Bereich zwischen 150 µm und 400 µm liegt. Durchbrüche mit diesen Abmessungen sind insbesondere auf Zellen mit einem Durchmesser von 20 µm abgestimmt.

Die Tiefe der Durchbrüche hängt vom vorgesehenen Einsatzzweck des erfindungsgemäßen Substrats ab. Sie wird dadurch begrenzt, daß unter physiologischen Bedingungen eine gute Nährstoffversorgung der zentralen Gewebszone im einzelnen Durchbruch (Kompartiment) gewährleistet sein muß. Unter diesem Gesichtspunkt erscheint eine Tiefe der Durchbrüche im Bereich zwischen 50 und 300 µm am besten geeignet. Für spezielle, unphysiologische Anwendungen erscheint eine Tiefe im Bereich zwischen 300 und 1000 µm besser geeignet. Solche Anwendungen sind beispielsweise Untersuchungen an Tumormodellen, bei denen eine zentrale nekrotische Zone bewußt erzeugt wird.

Besonders bevorzugt sind Substrate, deren Durchbrüche die Form eines sich in Richtung auf den Boden verjüngenden Pyramidenstumpfs aufweisen. Solche Substrate lassen sich mit den Methoden der mechanischen Mikrofertigung besonders einfach herstellen.

Durch eine geeignete Wahl der Breite der Stege auf der freien, nicht durch den Boden abgedeckten Seite des plattenförmigen Körpers läßt sich eine definierte Gewebsschicht auf kleinstem Raum herstellen. Hierbei wachsen die Zellen aus den Durchbrüchen heraus und verbinden sich zu einem das ganze Substrat überdeckenden Gewebeverband, der selbst keine störenden Stützstrukturen mehr enthält. Unter diesem Gesichtspunkt soll die Breite der Stege im Bereich zwischen 15 und 115 µm liegen.

Beim erfindungsgemäßen Substrat ist der Boden an sich nur während der Anlage der Zellkultur erforderlich. Da sich die Zellen, bedingt durch die erfindungsgemäße Wahl der Abmessungen, an den Wänden der Durchbrüche verankern, kann es insbesondere für eine mikroskopische Kontrolle der Zellkultur vorteilhaft sein, wenn der Boden abnehmbar ist. Im Gegensatz zum eingangs beschriebenen Substrat trägt hierbei nicht der Boden, sondern der plattenförmige, mit den Durchbrüchen versehene Körper die Zellkultur.

Als Boden kann eine mikroporöse, nur für Flüssigkeiten, nicht dagegen für einzelne Zellen durchlässige Platte, etwa eine keramische Fritte, oder eine eine entsprechende Kunststoffmembran eingesetzt werden. Eine gitterartige Bodenplatte mit definierten, durch Wände abgetrennte Öffnungen wird jedoch bevorzugt. Die Öffnungen können dabei so gewählt werden, daß einzelne Zellen zurückgehalten werden.

In jedem Fall wird der Boden gemäß der Erfindung so ausgelegt, daß die eingebrachten Zellen prinzipiell nur unwesentlich oder besser überhaupt nicht anheften können.

Dies kann beispielsweise dadurch erreicht werden, daß der Boden keine freie, ebene Fläche aufweist, die der Größe der einzelnen Zelle.entspricht. Wird beispielsweise eine keramische Fritte als Boden eingesetzt, soll die Porosität der Fritte entsprechend gewählt werden. Weiterhin kann der Boden aus einer Platte oder Folie bestehen, die aus einem solchen Material besteht, das für die Anheftung von Zellen ungeeignet ist.

Bei den bekannten, als Boden für Zellsubstrate einsetzbaren Platten oder Folien liegen die Größen der freien Flächen zwischen den Poren im allgemeinen innerhalb einer bestimmten Bandbreite. Ein gleichmäßigerer Boden besteht aus einem regelmäßigen Gitter mit durch Wände voneinander getrennten Öffnungen. Ein solches Gitter läßt sich ebenfalls mit den Methoden der Mikrofertigung (mechanische Mikrofertigung, Röntgentiefenlithographie, andere Lithographieverfahren etc.) herstellen.

Besonders bevorzugt sind solche gitterartigen Bodenplatten, die den Zellen bei der Anlage der Zellkultur prinzipiell keine ausreichende Fläche zum Verankern bieten. Deshalb werden die Wände des vorzugsweise eingesetzten Gitters maximal 20 µm, etwa 1 bis 20 µm dick und die lichte Weite der Öffnungen maximal 5 µm, etwa 1 bis 5 µm groß gewählt. Die untere Grenze der Abmessungen eines solchen Gitters wird meist durch das Herstellungsverfahren begrenzt sein. Vorzugsweise ist im Interesse der ungehinderten Zufuhr von Nährlösung und des ungehinderten Abtransports von Stoffwechselprodukten der Flächenanteil der Öffnungen in dem Boden größer als der Flächenanteil der Stege.

Es ist vorteilhaft, wenn das erfindungsgemäße Substrat aus einem durchsichtigen Material gefertigt ist. Als durchsichtiges Material ist insbesondere Polymethylmethacrylat (PMMA) geeignet. Substrate aus PMMA können mit den Methoden der Mikrofertigung einerseits einfach hergestellt werden; andererseits besitzt PMMA gewebefreundliche Eigenschaften.

Mikroskopische Untersuchungen können vereinfacht werden, wenn die Bodenplatte nach der Anlage der Zellkultur vom Gitter gelöst werden kann. Für den Fall, daß das Substrat aus dem plattenförmigen Körper und einem lösbaren Boden aufgebaut ist, reicht es für mikroskopische Beobachtungen aus, wenn nur der plattenförmige Körper aus einem durchsichtigen Material gefertigt ist.

Für manche Anwendungen können die Innenwände der Durchbrüche und die freien Flächen des Bodens in der Weise beschichtet werden, daß Zellen nicht anheften. In diesem Fall eignet sich das erfindungsgemäße Substrat außer zur Bildung definierter Gewebsschichten auch zur Erzeugung multizellulärer Sphäroide von definierter und sehr einheitlicher Größe. Eine hierfür geeignete Beschichtung wird beispielsweise durch eine Silikonisierung hergestellt. Bei einem solchen Substrat kommt es zu einer freien Aggregation der eingebrachten Zellen in den einzelnen Durchbrüchen (Kompartimenten). Die lichte Weite der Kompartimente bestimmt dann den Durchmesser der so gebildeten Sphäroide.

Das erfindungsgemäße Substrat stellt beispielsweise einen plattenförmigen Körper dar, der in der Art einer Gitterstruktur quadratische Vertiefungen enthält, die dicht nebeneinander angeordnet sind und sich in Form eines Pyramidenstumpfs nach unten verjüngen. Auf den Grundflächen der Vertiefungen sind weitere, wesentlich kleinere pyramidenförmige Vertiefungen angebracht, wobei die Spitzen der kleineren pyramidenförmigen Vertiefungen den plattenförmigen Körper durchstoßen. Bei dieser Ausführungsform sind der plattenförmige Körper und der Boden in einem Bauteil verbunden.

Eine weitere Ausführungsform des erfindungsgemäßen Substrats besteht darin, daß ein durchbrochener plattenförmiger Körper auf eine mikroporöse, insbesondere eine gitterförmige Bodenplatte aufgesetzt ist.

Durch dreidimensionale Kultivierung der Zellen im erfindungsgemäßen Substrat erfolgt Zelldifferenzierung zusätzlich zu den Substratkontakten über Zell-zu-Zell-Interaktionen. Neben der mikroskopischen Beobachtung können Serien-Dünnschnitte des mit Zellen gefüllten Substrates angefertigt werden, ein für die Anwendung histologischer und histochemischer Methoden entscheidender Vorzug.

Das erfindungsgemäße Substrat kann in einen Objektträger ebenso wie in ein Medium-Zirkulationssystem integriert werden. Bei mehrlagiger randdichter Ausfüllung mit Zellen stellt das Substrat eine Art Gewebefilter dar, wodurch zwei physiologische Halbräume oberhalb und unterhalb desselben definiert werden. Durch unterschiedliche Mediumbeschickung im Zirkulationssystem bilden sich vertikal gerichtete Stoffwechselgradienten in der Kultur aus, die auch durch eine zusätzliche Beschichtung der Gitterelemente (z. B. mit Komponenten der Extrazellularmatrix im Zuge zellspezifischer Anwendungen) nicht blockiert oder behindert werden.

Das erfindungsgemäße Substrat stellt eine stabile Stützstruktur für Zellen dar und ermöglicht die Herstellung von definierten Schichten aus verschiedenen Zelltypen. Durch die hierbei auftretenden heterotypischen Zellinteraktionen kann es zu weiteren Differenzierungssvorgängen kommen. Mit solchen Systemen können beispielsweise zellspezifische Transportprozesse studiert werden. Speziell könnten die in vitro nur schwer restituierbaren Bedingungen des Organsystems Haut näher untersucht werden, indem ein Halbraum von Luft anstelle von Medium durchströmt wird.

Eine solche "organnahe" Kulturtechnik auf der Basis des erfindungsgemäßen Substrates ist auf eine große Zahl verschiedenartiger Zelltypen anwendbar. Sie kann für Fragestellungen aus zahlreichen biomedizinischen Gebieten eingesetzt werden, bei denen ein Höchstmaß an Zelldifferenzierung erforderlich ist:
- Für toxikologische Untersuchungen zur Erzielung wirklichkeitsnaher Ergebnisse.
- Bei der Entwicklung von Arzneimitteln: Wirkungs- und Abbautests.
- In der Grundlagenforschung.
- Als Alternative zum Tierexperiment, vor allem bei der Untersuchung komplizierter Organsysteme (z. B. Leber oder Haut).

Die Erfindung wird im folgenden anhand von Figuren und Ausführungsbeispielen näher erläutert.

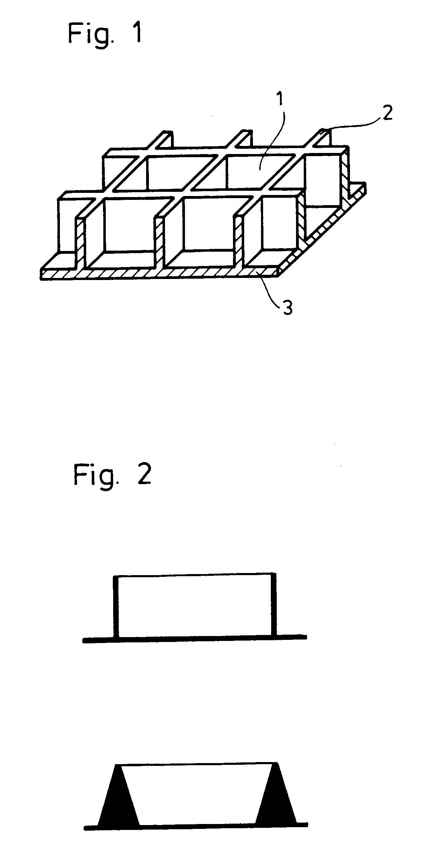

Fig. 1 zeigt eine Ausführungsform mit quaderförmigen Vertiefungen 1, die durch senkrechte Stege 2 voneinander getrennt und nach unten durch eine poröse Bodenplatte 3 abgeschlossen sind.

Fig. 2 zeigt die Ausführungsform gemäß Fig. 1 und eine abgewandelte Ausführungsform mit V-förmigen Stegen im Querschnitt.

Die Fig. 3 bis 5 zeigen verschiedene Bearbeitungsschritte eines Formeinsatzes, mit dem eine Ausführungsform des erfindungsgemäßen Substrats abgeformt werden kann.

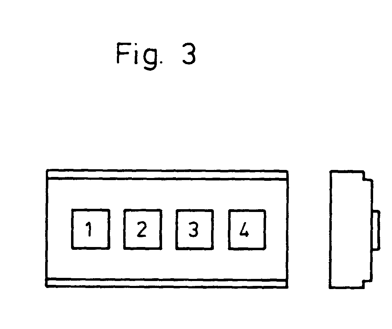

Fig. 3 stellt den vorbearbeiteten Formeinsatz dar.

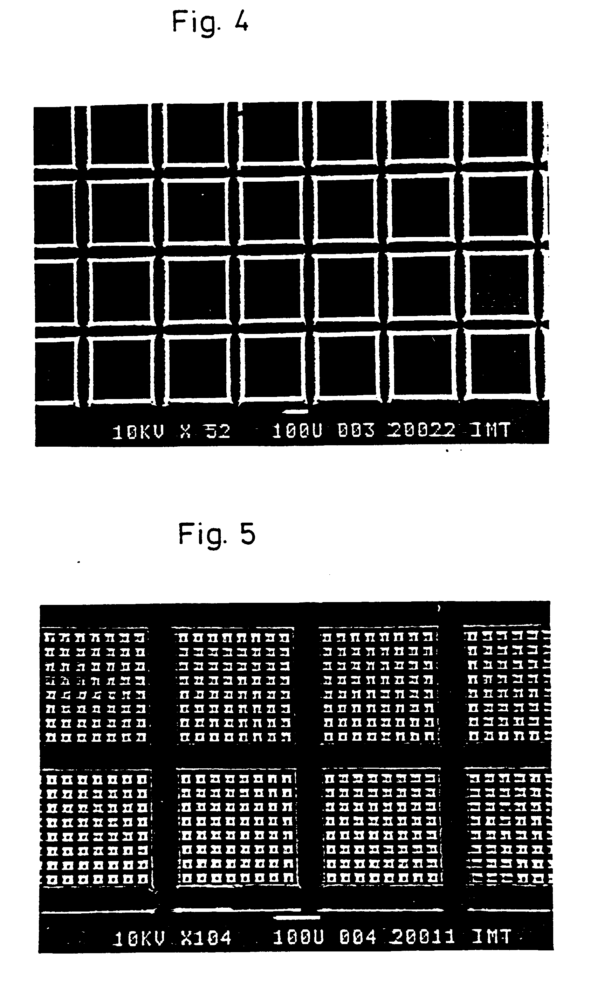

In Fig 4 ist der grobstrukturierte Formeinsatz dargestellt.

Fig. 5 zeigt den feinstrukturierten Formeinsatz.

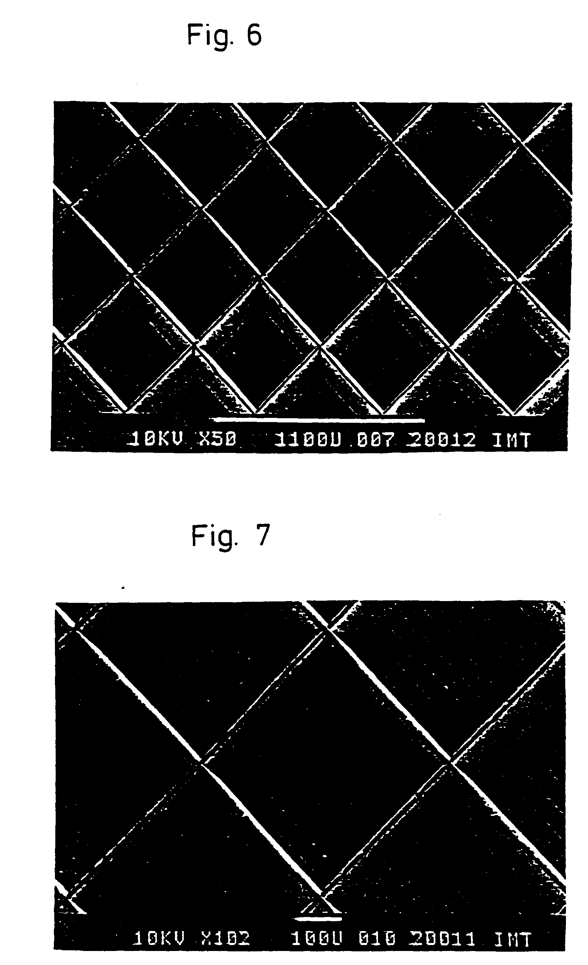

Fig. 6 zeigt mit dem grobstrukturierten Formeinsatz abgeformte Stege aus PMMA.

Fig. 7 zeigt mit dem feinstrukturierten Formeinsatz abgeformte Stege mit kleinen Einsenkungen am Grund der durch die Stege getrennten Durchbrüche, aus denen die Öffnungen hergestellt werden.

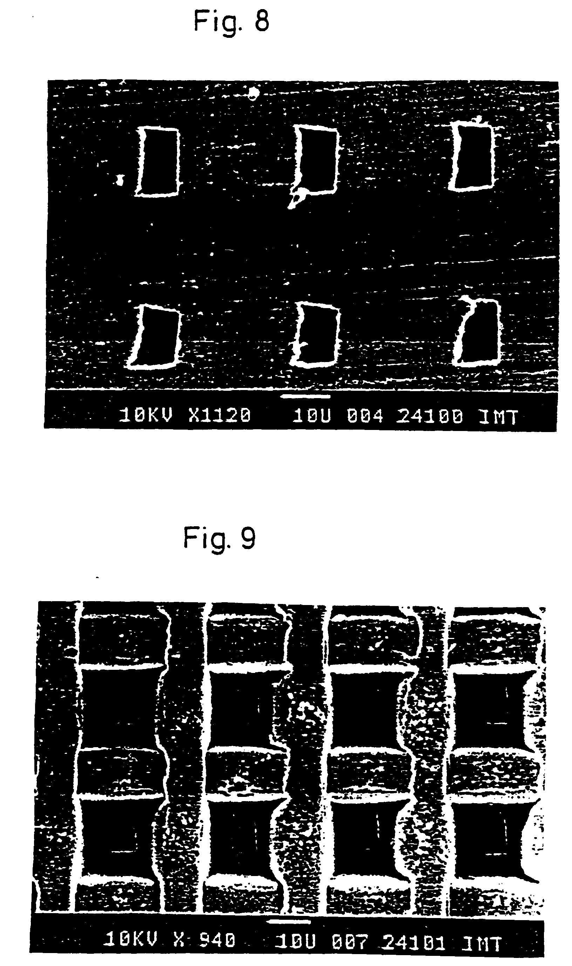

Fig. 8 zeigt die durch Fräsen bearbeitete Unterseite des Substrats mit den Öffnungen.

Fig. 9 stellt die Oberseite des in Fig. 8 gezeigten Substrats dar.

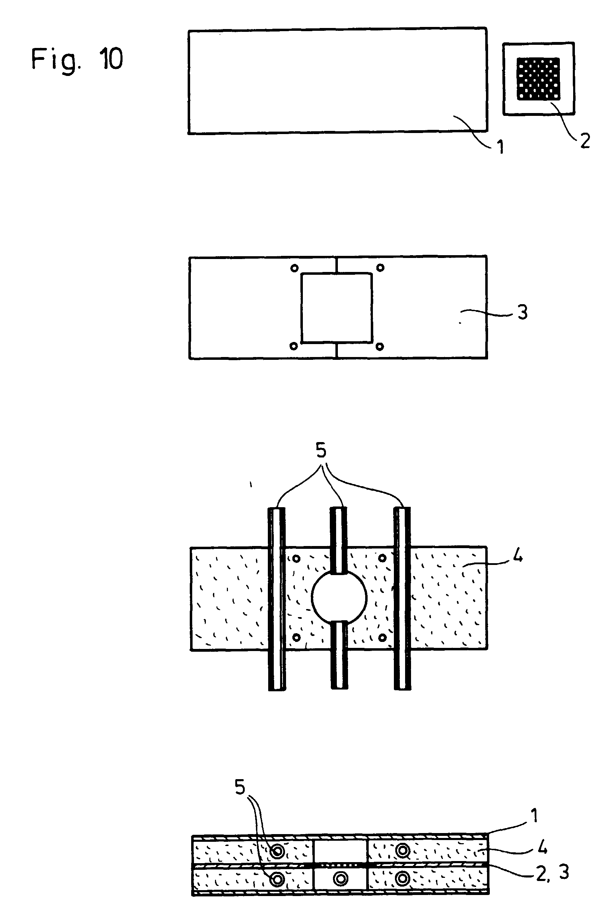

Fig. 10 zeigt einen Mikroskop-Objektträger, in den das Substrat integriert ist.

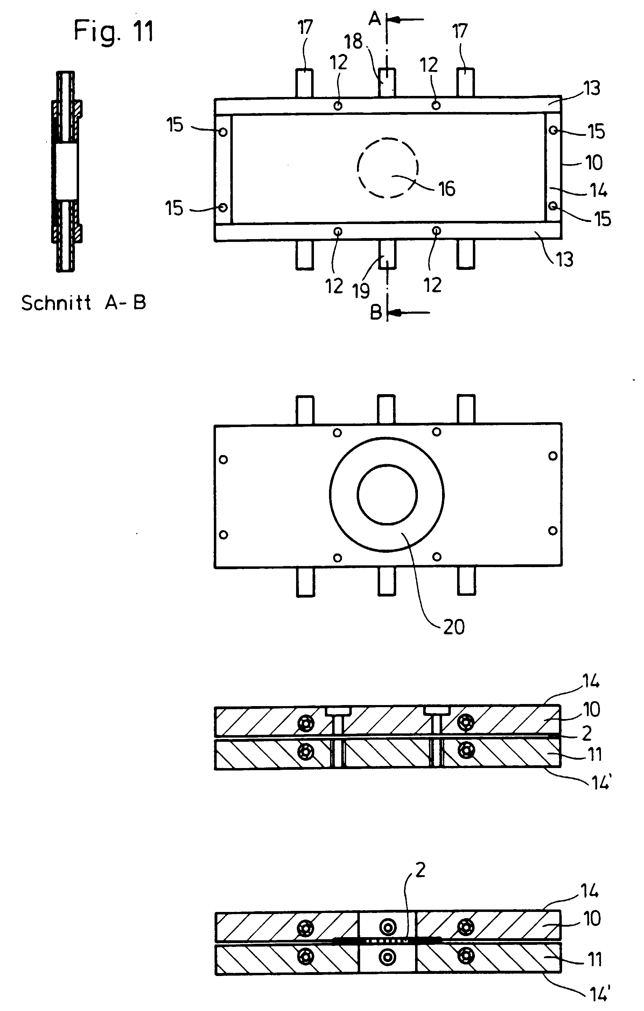

Fig. 11 zeigt einen weiteren Mikroskop-Objektträger mit integriertem Substrat.

### Beispiel 1

### Herstellung einer Ausführungsform des erfindungsgemäßen Substrats

Die erfindungsgemäßen Substrate können durch Röntgenlithographie-Verfahren oder durch Abformverfahren hergestellt werden. Im folgenden wird anhand der Fig. 3 bis 8 ein Abformverfahren beschrieben, mit dem auf einfache Weise eine Vielzahl der erfindungsgemäßen Substrate in Serie hergestellt werden können.

Zur Abformung eines Substrats aus Kunststoff wird ein Formeinsatz benötigt, in den die Positiv-Strukturen der Durchbrüche und Öffnungen des mikrostrukturierten Substrats eingebracht sind.

Fig. 3 zeigt einen vorbearbeiteten Formeinsatz mit vier erhabenen Plateaus von 10 mm x 10 mm in den Bereichen 1 bis 4. Die Höhe der Plateaus beträgt 1 mm. In jedes Plateau werden mit einem keilförmig profilierten Diamanten kreuzweise Nuten derart eingebracht, daß im Rastermaß 400 µm quadratische Netze aus 280 µm tiefen Gräben mit dreieckigem Querschnitt entstehen. Dadurch bleiben Pyramidenstümpfe mit Deckflächen von 300 µm x 300 µm stehen. Diese grobstrukturierte Form des Formeinsatzes ist in Fig. 4 dargestellt.

Danach werden in den Bereichen 1 bis 4 in die Pyramidenstümpfe durch erneute kreuzweise Bearbeitung mit feineren Diamanten eine Substruktur eingebracht: Die Deckfläche eines jeden Pyramidenstumpfs wird im Raster 40 µm durch 8 x 8 Mikropyramidenstümpfe strukturiert. Die Höhe der Mikropyramidenstümpfe liegt bei 80 µm, die Deckflächen messen einige 10 µm². Das Ergebnis dieses Bearbeitungsschritts ist in Fig. 5 dargestellt.

Der auf diese Weise bearbeitete Formeinsatz wird mit Hilfe einer Spritzgießmaschine abgeformt. Fig. 6 und 7 zeigen Grob- und Feinstruktur des in PMMA abgeformten Werkzeugs.

In einem abschließenden Bearbeitungsschritt wird das unter den abgeformten Strukturen befindliche Material entfernt; durch diesen Schritt werden die Öffnungen am Boden des Substrats freigelegt. Hierzu wird das Substrat auf einer Vakuumspannvorrichtung fixiert und solange Material von der Unterseite des Substrats entfernt, bis die Öffnungen bzw. Poren des Bodens offen zutage treten. Über das Maß des Abtrags läßt sich die Dicke des Bodenteils und der minimale Porendurchmesser steuern. Das Ergebnis dieses Verfahrensschritts ist in den Fig. 8 (Unterseite des Substrats) und 9 (Oberseite des Substrats) dargestellt. Wird der Boden des Substrats vollständig entfernt, läßt sich ein Kunststoffgitter aus 100 µm breiten Stegen mit Maschenweiten von 300 µm herstellen, das als plattenförmiger Körper dienen kann.

### Beispiel 2

### Herstellung eines Mikroskop-Objektträgers

Ein gemäß Beispiel 1 hergestelltes Substrat 2 mit einer benutzbaren Fläche von 10 mm x 10 mm wird, wie in Fig. 10 gezeigt, mit zwei Glasobjektträgerplättchen 1, Justierscheiben 3 und Zwischenscheiben 4, die Thermostatisierungs- und Nährlösungskanäle 5 enthalten, zu einem Objektträger vereinigt.

Die äußeren Kanäle dienen der Thermostatisierung; über die mittleren Kanäle wird Nährlösung zu- und abgeleitet.

### Beispiel 3

### Herstellung eines weiteren Mikroskop-Objektträgers

Der Mikroskop-Objektträger besteht aus zwei nahezu identischen Zwischenscheiben 10, 11, zwischen denen ein gemäß Beispiel 1 hergestelltes Substrat 2 flüssigkeitsdicht eingespannt werden kann. Die obere 10 und untere 11 Zwischenscheibe sind bis auf den Unterschied identisch, daß die obere seitlich vier Bohrungen 12 und die untere an den entsprechenden Stellen vier Gewinde (nicht dargestellt) enthält, durch die mittels Schrauben das dazwischenliegende Substrat 2 eingeklemmt wird. Die Zwischenscheiben sind aus Messing gefertigt und oberflächlich durch eine galvanisch aufgebrachte Schicht aus Rhodium für die Gewebekultur veredelt. Die jeweilige Außenfläche ist mit einer rechteckigen Einfräsung der Breite versehen, daß Sie ein handelsübliches Mikroskop-Objektträgerplättchen 14 aufnehmen kann, das durch jeweils zwei an den kurzen Außenkanten befindliche Kunststoffschrauben (in den Bohrungen 15) arretiert wird. Die Zwischenscheiben sind mit einer zentralen Bohrung 16 versehen, durch die eine optische Beobachtung der Zellen im Substrat möglich ist.

Auf der Innenseite befindet sich eine kreisförmige Einfräsung 20, die der paßgenauen Aufnahme des Substrats dient.

Zwei äußere durchgehende Rohre 17 (Innendurchmesser 3 mm, Außendurchmesser 4 mm) können über den Anschluß an ein externes Wasserbad mit Warmwasser zur Temperierung durchströmt werden. Mittlere Rohre 18, 19 ragen von gegenüberliegenden Seiten in die zentrale Bohrung 16 hinein. Durch diese wird frisches Nährmedium durch den Hohlraum geleitet, der an der mittleren Fläche durch das Substrat 2 und an der äußeren Fläche durch das jeweilige Objektträgerplättchen 14 begrenzt ist. Dadurch entstehen im zusammengebauten Zustand zwei Hohlräume, die von unterschiedlichen Nährmedien durchströmt werden können.

Das gesamte System ist somit von oben nach unten symmetrisch durch die folgenden Komponenten aufgebaut:

Objektträgerplättchen 14, verschraubt an die obere Zwischenscheibe 10, verschraubt unter Zwischenlage des Substrats mit der unteren Zwischenscheibe 11, an der ein weiteres Objektträgerplättchen 14' verschraubt ist.

Mit den gemäß Beispiel 1 hergestellten Substraten wurden im folgenden biologische Versuche durchgeführt, um die Kultivierung dreidimensionaler Zellkulturen zu demonstrieren.

### Beispiel 4

### Beschickung des Substrats mit Zellen

Die Experimente wurden mit zwei permanenten Zellinien der Maus durchgeführt: L- und SV40-3T3-Zellen. Es kamen durchweg Standard-Kulturmedien zum Einsatz.

Zunächst wurden die Substrate durch Co-Gammabestrahlung mit ca. 100 Gy sterilisiert und in eine trockene Petrischale überführt. Auf die 1 cm² große Gitterfläche wurden 300 bis maximal 400 µl einer konzentrierten Suspension von Zellen bzw. Sphäroiden (Zellaggregate) aufgetragen. Durch die Kapillarwirkung kam es zu einem raschen Eindringen in die Vertiefungen des Substrats innerhalb von ca. einer Minute unter vollständiger Mitnahme der Zellen oder Sphäroide. Nach ca. 15 Minuten im Brutschrank bei 37 °C wurde die Petrischale vorsichtig mit Medium gefüllt, wobei zwei Varianten erprobt wurden. Einmal wurde die Gitterstruktur unterschichtet, so daß sie auf dem Mediumfilm schwamm. Alternativ wurde sie überschichtet. In beiden Fällen etablierte sich ein vollständiger Kontakt des Mediums mit den Zellen im Substrat; beide Varianten erwiesen sich als gleichwertig in Bezug auf die weitere Kultivierung der Zellen im Brutschrank. Die Beschickung des Substrats ist insgesamt problemlos; auf eine zusätzliche vorsichtige Zentrifugierung zur Verbesserung des Eindringens der Zellen bzw. Sphäroide kann im allgemeinen verzichtet werden.

### Beispiel 5

### Zur Gewebefreundlichkeit des verwendeten Kunststoffs PMMA

Ein weiteres Testziel bestand in der Untersuchung der Gewebefreundlichkeit des Substratmaterials. Als Kriterium wurde hier die Ausbildung von mikroskopisch sichtbaren Kontakten mit den Wänden herangezogen. Es zeigte sich, daß es innerhalb weniger Stunden zur Anheftung der Zellen an den Wänden der Vertiefungen des Substrats kommt. Zusammen mit Vitalitätstests und weiteren Details der Zellwechselwirkung mit den Gitterwänden wird damit die Gewebeverträglichkeit des Substratmaterials PMMA und der Geometrie der Substratstruktur demonstriert. Die beobachtete hohe Affinität der Zellen zum Material wird möglicherweise durch die Vorbestrahlung zur Sterilisierung begünstigt. Die hierbei auftretenden Oberflächenveränderungen (Veränderung des Ladungsmusters) können die Ausbildung von Zellkontakten erleichtern.

### Beispiel 6

### Vitalität und Wachstumsverhalten der Zellen

Das Testziel bestand zum einen darin, etwaige zellschädigende Wirkungen bei der Langzeitkultur im Substrat zu identifizieren. Zum andern sollte das Wechselwirkungsmuster der Zellen mit den Gitterwänden und ihre morphologische Differenzierung untersucht werden. Um zellschädigende Wirkungen zu identifizieren, wurden Zellen und Sphäroide in den Gitterstrukturen über mehr als 2 Wochen kultiviert. Die Nährstoffversorgung erfolgte durch Mediumwechsel alle 2 bis 3 Tage. Während dieser Zeit blieb die Kultur steril, die Zellen lebens- und teilungsfähig. Beim Abbruch der Versuchsreihen waren noch alle Zellen vital (Nachweis durch Trypanblau-Ausschlußtest) und teilungsfähig (Klonogenitätstest). Von dem Kultursystem gehen damit, selbst bei Langzeitkultur, keinerlei zellschädigende Wirkungen aus.

Zur Herstellung und Kultur mehrlagiger Zellschichten in den Vertiefungen des Substrats kann man von Einzelzellen oder bereits fertigen Sphäroiden ausgehen. Die Wahl wird in der Praxis nach den Eigenschaften der verwendeten Zellarten, z. B. nach ihrer proliferativen Kapazität, getroffen.

Bei der Einbringung von Einzelzellen kommt es zunächst zur Anheftung an die Wände der Vertiefungen und zwar meist selektiv etwa in der Mitte der Wände, d. h. auf halber Wandhöhe. Eine Besiedlung des porösen Bodens der Vertiefungen findet nur in geringem Umfang statt. Von den Wänden ausgehend vermehren sich die Zellen ohne mechanische Unterstützung zur Mitte der Vertiefungen hin. Es entsteht ein mehrlagiges dichtes Zellgeflecht. Dieses Verhalten hängt in gewissem Umfang vom Typus der eingesetzten Zellen ab und war bei den L-Fibroblasten besonders ausgeprägt.

Zur Untersuchung des Verhaltens von Sphäroiden wurden die Vertiefungen mit SV40-3T3-Sphäroiden mit einem mittleren Durchmesser von 200 µm beschickt. Sie waren damit kleiner als die Vertiefungen, so daß ihr Wachstumsverhalten verfolgt werden konnte. Sie lagerten sich zunächst jeweils an eine Wand der Vertiefungen an. Die Zellvermehrung führte zu einer Volumenzunahme der Sphäroide, so das diese nach wenigen Tagen die Vertiefungen randdicht ausfüllten. Die Kontaktbildung der äußeren Sphäroidzellen mit den Substratwänden und damit die Ausbildung eines mehrschichtigen gewebeartigen Zellverbands konnte mikroskopisch nachgewiesen und verfolgt werden.

### Beispiel 7

### Ablösung der Zellen zur Analyse; Wiederverwendbarkeit der Substrate

Die in den Vertiefungen verankerten Zellen können mit Hilfe einer enzymatischen Behandlung mit Trypsin herausgelöst und weiter analysiert werden. Die lichtmikroskopische Inspektion zeigt, daß die Zellen dabei quantitativ aus den Vertiefungen entfernt werden. Auch eine einwöchige Inkubation des geleerten Substrats mit frischem Kulturmedium ergab, daß keine Zellen in den Vertiefungen verblieben. Gleichzeitig konnte damit die Aufrechterhaltung der Sterilität dokumentiert werden. Auf diese Art "gereinigte" Substrate sind wiederholt einsetzbar.

## Patentansprüche

1. Substrat für Zellkulturen bestehend aus
a) einem plattenförmigen Körper mit gitterartig angeordneten, gegeneinander durch Stege getrennten Durchbrüchen und
b) einem für Flüssigkeiten, nicht jedoch für Zellen durchlässigen Boden, der die Durchbrüche einseitig verschließt,
dadurch gekennzeichnet, daß
c) die lichte Weite der Durchbrüche im Bereich zwischen 50 µm und 1000 µm liegt und
d) ein Boden gewählt wird, der die Anheftung von Zellen verhindert.

2. Substrat nach Anspruch 1, dadurch gekennzeichnet, daß
die lichte Weite der Durchbrüche im Bereich zwischen 150 µm und 400 µm liegt.

3. Substrat nach Anspruch 1 oder 2, dadurch gekennzeichnet, daß
die Tiefe der Durchbrüche für physiologische Zellkulturen im Bereich zwischen 50 µm und 300 µm liegt.

4. Substrat nach Anspruch 1 oder 2, dadurch gekennzeichnet, daß
die Tiefe der Durchbrüche für unphysiologische Zellkulturen im Bereich zwischen 300 µm und 1000 µm liegt.

5. Substrat nach einem der Ansprüche 1 bis 4, dadurch gekennzeichnet, daß
die Breite der Stege auf der freien, nicht durch den Boden abgedeckten Seite des plattenförmigen Körpers im Bereich zwischen 15 µm und 115 µm liegt.

6. Substrat nach einem der Ansprüche 1 bis 5, dadurch gekennzeichnet, daß
der Boden ein Gitter mit durch Wände voneinander getrennten öffnungen darstellt.

7. Substrat nach Anspruch 6, dadurch gekennzeichnet, daß
die Wände des Gitters maximal 20 µm dick sind und die öffnungen eine lichte Weite von maximal 5µm aufweisen.

8. Substrat nach einem der Ansprüche 1 bis 7, dadurch gekennzeichnet, daß
als Werkstoff ein durchsichtiges Material eingesetzt wird.

9. Substrat nach Anspruch 8, dadurch gekennzeichnet, daß
das Substrat in einen Mikroskop-Objektträger integriert ist.

## Claims

1. Substrate for cell cultures, comprising
a) a plate-like body having openings which are disposed in the form of a lattice and are separated from one another by webs, and
b) a base, which is permeable to fluids, but not to cells, and seals the openings on one side,
characterised in that
c) the inside width of the openings lies in the range of between 50 µm and 1000 µm, and
d) a base is selected, which prevents the attachment of cells.

2. Substrate according to claim 1, characterised in that the inside width of the openings lies in the range of between 150 µm and 400 µm.

3. Substrate according to claim 1 or 2, characterised in that the depth of the openings for physiological cell cultures lies in the range of between 50 µm and 300 µm.

4. Substrate according to claim 1 or 2, characterised in that the depth of the openings for non-physiological cell cultures lies in the range of between 300 µm and 1000 µm.

5. Substrate according to one of claims 1 to 4, characterised in that the width of the webs on the free side of the plate-like body not covered by the base lies in the range of between 15 µm and 115 µm.

6. Substrate according to one of claims 1 to 5, characterised in that the base represents a lattice having apertures separated from one another by walls.

7. Substrate according to claim 6, characterised in that the maximum thickness of the walls of the lattice is 20 µm, and the apertures have a maximum internal width of 5 µm.

8. Substrate according to one of claims 1 to 7, characterised in that a transparent material is used as the material.

9. Substrate according to claim 8, characterised in that the substrate is incorporated in a microscope slide.

## Revendications

1. Substrat pour cultures de cellules, se composant :
a) d'un corps en forme de plaque avec des ouvertures disposées en type grillage, séparées les unes contre les autres par des entretoises et,
b) d'un fond pour des liquides, mais imperméable pour les cellules, qui obture d'un côté les ouvertures,
caractérisé en ce que
c) la largeur libre de passage des ouvertures se situe dans la zone comprise entre 50 µm et 1000 µm et,
d) on choisit un fond, qui empêche la fixation des cellules.

2. Substrat selon la revendication 1,
caractérisé en ce que
la largeur libre de passage des ouvertures se situe dans la zone comprise 150 et 400 µm.

3. Substrat selon les revendications 1 ou 2,
caractérisé en ce que
la profondeur des ouvertures pour des cultures de cellules physiologiques se situe dans la zone comprise entre 50 µm et 300 µm.

4. Substrat selon les revendications 1 ou 2,
caractérisé en ce que
la profondeur des ouvertures pour des cultures de cellules physiologiques se situe dans la zone de 300 µm à 1000 µm.

5. Substrat selon une des revendications 1 à 4,
caractérisé en ce que
la largeur des entretoises sur le côté libre, non couvert par le fond du corps en forme de plaque se situe dans la zone comprise entre 15 µm et 115 µm.

6. Substrat selon une des revendications 1 à 5,
caractérisé en ce que
le fond représente une grille avec des ouvertures séparées les unes des autres par des parois.

7. Substrat selon la revendication 6,
caractérisé en ce que
les parois de la grille ont une épaisseur maxima de 20 µm et les ouvertures une largeur libre de passage maxima de 5 µm.

8. Substrat selon une des revendications 1 à 7,
caractérisé en ce que
comme matériau, on met en oeuvre un matériau transparent.

9. Substrat selon la revendication 8,
caractérisé en ce que
le substrat est intégré dans une lame porte-objet de microscope.
